# EUROPEAN PATENT APPLICATION

(11) **EP 1 579 804 A1**
(43) Date of publication of application: **28.09.2005**
(21) Application number: 05075674.1
(22) Date of filing: 22.03.2005
(51) Int. Cl.: A61B 5/11, A61B 5/01

(54) **Detecting a physical condition of health of an animal**

(30) Priority: 22.03.2004 DK 200400456
(71) Applicant: Infradan ApS, 7870 Roslev (DK)
(72) Inventor: Tambo, Jan, 8751 Gedved (DK)
(74) Representative: Norris, Timothy Sweyn

(57) **Abstract**

The present invention relates to a method of detecting a physical condition of health of an animal, for example a mother animal breastfeeding at least one corresponding young animal, and of providing one or more signals representing this condition so as to enable a farmer to take one or more appropriate actions based on the condition of the animal. The invention also relates to an apparatus for implementing the method.

## Description

### FIELD OF THE INVENTION

The present invention relates to methods of detecting a physical condition of health of an animal and of providing one or more signals representing this condition so as to enable a farmer to take one or more appropriate actions based on the condition of the animal. The invention also relates to apparatus operable to implement the methods.

### BACKGROUND TO THE INVENTION

For various reasons, it is found that contemporary piglet mortality is still relatively high, despite efforts having been made to decrease such piglet mortality. Piglets die for numerous reasons. One plausible reason for high piglet mortality may be that a sow is capable of giving birth to an increased number of piglets and that the space contemporarily provided for such increased number of piglets has not been increased in a correlated manner. A reason why piglets die may, for example, be that the sow places herself on top of one or more of the piglets waiting to be nourished from the sow's udder as she lies down. Various efforts have been made:
(a) to decrease a risk of piglets being present in proximity of the sow or under the sow in certain situations; and
(b) for the piglets to be present in the proximity of the sow for no longer a time interval than necessary to be nourished.
These various efforts will now be described.

It is known to monitor a physical health of an animal by monitoring the animal itself. Such monitoring allows identification of any sign indicative of the physical condition of the animal in order to detect any unhealthy condition of the animal as early as possible. Detection of any unhealthy condition enables necessary precautions to be taken for curing the animal or at least for minimising the severity of any such unhealthy condition.

In a published United States patent no. US 5,921,205, there is described a farrowing station for constraining the movement of a sow so that she is at liberty to stand in the station and to lie down within the station, thereby enabling her to nurse her litter of piglets. A special transportable floor construction is provided for preventing the piglets from dying when the sow lies down.

In a published United Kingdom patent no. GB2117620, there is described a pig farrowing crate comprising a blowing arrangement, for example a fan, operable to blow air into a lower part of the crate below an udder of a sow standing in the crate. Moreover, a sensing arrangement is provided to sense the presence of a standing sow so that the air blowing arrangement can be activated only when a standing sow is detected. Thus, piglets of the sow are deterred from standing in a hazardous position beneath the sow's udder.

The published patents nos. US 5,921,205 and GB2117620 seek to reduce the risk of the sow overlaying her piglets by mechanically removing the piglets as the sow lays down, and by blowing air under the sow when she is standing to prevent the piglets from being under the sow. However, the aforementioned published patents do not disclose a sufficiently low cost, and easy-to-maintain approach, for improving the living conditions of at least the piglets and their corresponding sow.

Various attempts have been made both to improve weight gain and to decrease mortality for improving living conditions for at least the piglets. An approach to enhance living conditions is described in an International PCT application no. WO 03/056907 which discloses a method and arrangement for controlling a temperature in an area of an animal pen, for example a nest of piglets. Preferable, a measurement of a surface temperature within the nest is utilized, for example provided by way of infrared temperature measurement. The measure of surface temperature provides a measure of a surface temperature of the piglets, thereby enabling the temperature of the nest to be controlled so as to attract the piglets in an optimised manner, thereby decreasing mortality and improving piglet weight gain.

The arrangement and method described in the application WO 03/056907 seek to reduce a risk of a sow overlaying her piglets by regulating a surface temperature for the piglets in the nest, such regulation employing a heat source, a temperature measuring device and an automatic controller for setting the temperature neither too high nor too low.
Moreover, such regulation employs a method of controlling the heat source in response to temperature measurement signals provided from the temperature measuring device by way of the automatic controller, thereby, for example, reducing the time in which piglets may be in a hazardous zone under or in proximity of their sow. However, the application WO 03/056907 does not seek to provide a solution of improving both the living conditions of piglets and their associated sows.

The present invention is aimed at addressing drawbacks associated with the aforementioned prior art.

### SUMMARY OF THE INVENTION

An object of the present invention is to provide one or more signals representing an ability of a sow breastfeeding her piglets to provide nourishment to the piglets, together with methods of interpreting these one or more signals; these signals are beneficially determined as early as possible and/or continuously during the time of life of the piglets.

A further object of the invention is to detect a disease or other illness of an animal, for example only in a situation where the disease or illness is difficult to detect from an animal itself.

The objects of the invention may be addressed by a method of detecting a physical condition of health of a mother animal breastfeeding at least one corresponding young animal,
said method comprising steps of
- monitoring one or more movements of said at least one young animal within an area;
- collecting positional data of said at least one young animal;
- processing said positional data to generate data representative of movement patterns of said at least one young, and
- analysing said data representative of movement patterns of said at least one young animal to determine the physical condition of health of said at least one young animal.

The invention is of advantage in that, by monitoring the movement patterns of the at least one young animal, it is possible to determine the physical condition of health without having to interfere with the at least one young animal itself, such interference including, for example weighing the at least one young animal, and attaching tags or other descriptive elements to the at least one young animal.

Optionally, in the method, the analysing step comprises a further step of:
- comparing said data representative of movement patterns of said at least one young animal with a reference movement pattern,
wherein said reference movement pattern is a movement pattern of a young animal during its time of life when breastfeeding by its mother animal when in a healtily state, or a movement pattern of said young animal corresponding to another time interval.

By making such a comparison, it is possible to compare the measured movement patterns with other movement patterns of the animals themselves, or to compare the movement patterns with known patterns of animals under influence of known factors. It is thereby feasible to determine promptly an unhealthy condition of a given animal without having previously monitored movement patterns of the given animal.

Optionally, the method further comprises steps of
- dividing said area being monitored into at least two zones, namely a feeding zone and a nesting zone, said feeding zone being a region wherein said at least one young animal is breastfed,
- comparing said movement patterns with said reference movement pattern to detect durations and frequencies said at least one young animal spends in at least one of said zones, and
wherein the durations and frequencies said at least one young animal spends in the at least one zone are indicative of the physical condition of health of at least said mother animal.

By detecting the durations and frequencies of the movement patterns of the mother animal and her at least one young animal, further knowledge of the physical condition of the animals may be obtained; for example, if the animals stay in the nesting zone for a relative long time, it is possible to determine whether or not the at least one young animal is well nourished.

Optionally, in the method, the movement patterns are determined by measuring at least one value within the area, said value being correlated to said at least one young animal during its time of life when breastfed by said mother animal. The measured values may be any value suitable for indicating the physical condition of the animals such as the frequency at which the animals enter and exit the area, the duration of stay of the animals in the area, the number of animals within the area, a temperature or a humidity in the area or any heat emission by the animals when staying in the area.

Optionally, in the method, the movement patterns are determined by measuring at least one value of a temperature within the nesting zone. The at least one value of a temperature may be used as an indication of the presence or non-presence of animals in the area and may also be used as an indication of the number of animals present in the area.

Yet more optionally, for example as a supplement to only measuring the temperature or as the only measurement made, the method may comprise a step of determining the movement patterns by measuring a value of power supplied to a heater arrangement operable to heat the nesting zone.

The power supplied to the heating arrangement may be used as an indication of a need to heat the area to achieve a desired temperature in the area, and as an indication of a presence of animals in the area causing a decreased requirement for power to be supplied to the heating arrangement, and as an indication of a non-presence of animals causing an increased need for power supply to the heating arrangement. For example, by measuring power supplied to the nesting zone, a change in heat supplied by the at least one young animal is indicated, and by this indication it is feasible to derive a measure of nourishment received by the at least one young animal.

More optionally, in the method, the movement patterns are determined by measuring at least one value of an infrared surface temperature of a surface within the nesting zone. In an example embodiment of the invention, an actual precise body surface temperature of a plurality of young animals within the nesting zone is used to provide an average temperature of the surface in the nesting zone.

According to a first embodiment of the invention, there is provided an apparatus for detecting a physical condition of health of a mother animal during its time of life when breastfeeding at least one corresponding young animal, said apparatus comprising
- a temperature measuring arrangement for measuring at least one temperature of a nesting zone, said nesting zone being disposed for receiving said at least one young animal;
- a processing and analyzing arrangement for processing and analyzing said at least one temperature measured by said temperature measurement arrangement, and for detecting a duration and frequency of said at least one young animal in said nesting zone and generating a corresponding output;
- a determining arrangement for determining from said output from said processing and analyzing arrangement at least one physical condition of health of said mother animal during its time of life when breastfeeding said at least one young animal or of said at least one young animal; and
- a signal generating arrangement coupled to said determining arrangement for providing at least one signal in response to said determination for providing an alarm indicative of a physically unhealthy condition of the mother animal and/or said at least one young animal.

According to a second embodiment of the invention, there is provided an apparatus for detecting a physical condition of health of a mother animal during its time of life when breastfeeding at least one corresponding young animal, said apparatus comprising:
- a power measuring arrangement for measuring power supplied to a heating device of a nesting zone, said nesting zone being disposed for accommodating said at least one young animal;
- a processing and analyzing arrangement for processing and analyzing said power measurement, for detecting a duration and frequency in which said at least one young animal is in the nesting zone;
- a determining arrangement for determining, based on the said detection, at least one physical condition of health of said mother animal during its time of life when breastfeeding said at least one young animal or of said at least one young animal being breastfed by the mother animal, and
- a signal generating arrangement coupled to said determining arrangement for providing at least one signal in response to said determination for providing an alarm indicative of a physically unhealthy condition of the mother animal and/or at least one young animal.

In an example embodiment of an apparatus, the apparatus comprises a temperature measurement arrangement including an infrared temperature measuring device for measuring a surface temperature in the nesting zone.

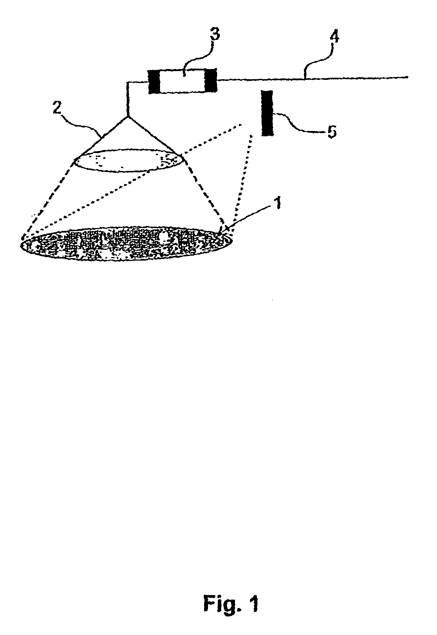

### DESCRIPTION OF THE DRAWING

Embodiments of the invention will now be described, by way of example only, with reference to the drawing wherein there is shown a nesting zone and a schematic view of main elements of an embodiment of an apparatus according to the invention.

### DESCRIPTION OF EMBODIMENTS OF THE INVENTION

In the drawing, there is shown a control, registration and transceiver unit (CRTU) 3 connected to a power source 4, an infrared average surface temperature measurement arrangement 5 and a heat source such as a heating lamp 2. The infrared temperature measurement arrangement 5 is operable to measure an average surface temperature of a nesting zone 1, namely with or without piglets being present in the nesting zone 1, and to provide the CRTU 3 with a measure of the average nesting zone surface temperature, for example an updated measure of average temperature each minute averaged over a period of one minute. The CRTU 3 regulates the power supplied from the power source 4 to the heating lamp 2 based on the surface temperature determined by the temperature measurement arrangement 5; the CRTU 3 is thereby operable to regulate to a set value representing a desired average surface temperature level in the nesting zone 1. This set value may be reset and/or changed by a user, for example a farmer, from a handheld device (not shown), from a central unit (not shown), of via a wireless connection from the CRTU 3. The CRTU 3 also comprises a power measurement arrangement for measuring power supplied to the heating lamp 2 necessary for providing the desired average surface temperature in the nesting zone 1.

The CRTU 3 comprises a registration arrangement for registering and storing a measured average power supplied to the heating lamp 2 together with an actual average surface temperature in the nesting zone 1; optionally, both average values are averaged over a one minute period. In this embodiment, every second of both these values are transmitted from a transceiver included in the CRTU 3 to the central unit. The values may also be wireless-transmitted to the handheld device every second minute or transmitted by the CRTU 3 to the handheld device in response to a request from the handheld device. The request can be generated by inputting a number identifying a given farrowing pen into the handheld device, or by scanning using the handheld device a barcode identifying the given farrowing pen.

An average of the measured average power supplied and an average surface temperature of the nesting zone 1 within an interval is provided by the CRTU 3. In this described embodiment of the invention, the interval is optionally three hours, but may be set to other durations. The average values for both the average surface temperature and the average power supplied of two succeeding intervals are compared; a signal is generated by the CRTU 3 in response to this comparison. This signal may be presented on the CRTU 3 to the user by way of a visual indication. Alternatively, of additionally, the signal may be transmitted to the aforesaid handheld device. Yet alternatively, or additionally, the signal may be communicated to the central unit or, for example via the central unit, as a contemporary Short Message Service (SMS) message to the farmer. SMS messages are contemporarily communicated between mobile telephones (cell phones).

When, in general, the average power supplied within a more recent 3 hour interval is less than the average power supplied in the first interval, piglets have been using the nesting zone 1. By the piglets producing energy within the nesting zone 1 and increasing the surface temperature of the nesting zone 1, no signal in such case is provided representing a physical condition of health of the sow, said condition being indicative of the sow being able to feed its piglets. In such a situation, the piglets produce energy in general and produce even further energy when well nourished by their corresponding sow; the piglets thus have a body surface temperature normally substantially higher than the surface temperature of the nesting zone 1 when devoid of piglets.

When, in general, the average power supplied within a more recent 3 hour interval is more than or substantially equal to the average power supplied in the first interval, the piglets have not been using the nesting zone 1 at all indicating that:
(a) the piglets have remained in proximity of the sow to fulfil their basic primary need for nourishment; or
(b) the piglets have not been using the nesting zone 1 more than the last interval; or
(c) the piglets have been using the nesting zone 1 but have not been provided with enough nourishment by the sow.
A signal indicative of the physical condition of the sow, said condition relating to an ability of the sow to feed her piglets, is provided; in the situation described in (a) to (c), the signal is indicative of the sow of this farrowing pen not, for the intervals concerned, being able to nourish its piglets to a desired degree.

When, in general, the average surface temperature within the last 3 hours interval, is higher than the average surface temperature in the first interval, the piglets have been using the nesting zone 1. By increasing the surface temperature of the nesting zone 1, no signal representing the physical condition of health of the sow is provided, said condition being indicative of the ability of a sow to feed its piglets.

When, in general, the average surface temperature within the last 3 hours interval, is lower than the average surface temperature in the first interval, the piglets have not been using the nesting zone 1 at all, indicating that the piglets have remained in the proximity of their sow to fulfil their basic need for nourishment. It may also or additionally be an indication of the piglets not having been using the nesting zone 1 more than within the last interval. It may also or additionally be an indication of the piglets having been using the nesting zone 1, but not having been provided enough nourishment by their sow. In all situations, a signal is provided representing the physical condition of the sow, said condition being indicative of an ability of the sow to feed her piglets. The signal potentially indicates that the sow of this farrowing pen may not, for the intervals concerned, be able to nourish its piglets to a desired degree of nourishment.

When using both the average power supplied and the average surface temperature, and especially when a comparison is made of changes in these average values, there can be derived therefrom a signal which more reliably is indicative of a physical condition of health. For example, the average surface temperature must be substantially lower and the power supplied must be substantially higher in the same comparison interval of time, before a signal representing the physical condition of the sow is provided, said condition being indicative of an ability of the sow to feed her piglets. The signal is potentially indicative that the sow of this farrowing pen is not, for the intervals concerned, able to nourish her piglets to a desired degree of nourishment.

In order to determine:
(a) whether or not the piglets use the nesting zone 1;
(b) a sequence in which the piglets move in and out of the nesting zone 1; and
(c) when each piglet moves in and out of the nesting zone 1,
a method is described in the foregoing which utilizes average surface temperature measurements and compares said temperature measurements for a given time interval for a certain time frame such as a week, such as a day, such as an hour, such as half an hour, such as five minutes or such as intervals down to one minute, and identifies the movements of the piglets as changes between the measured temperatures. In general, when the average surface temperature in the nesting zone 1 and/or the average power supplied to the nesting zone 1 fluctuates, this indicates that the nesting zone 1 is used by the piglets.

In an example embodiment of the invention, the central unit is operable to receive the average values every second minute and may provide a list of numbers representing farrowing pens. The farrowing pens on top of the list are optionally representative of pens whose piglets are oldest and whose heat sources use relatively most power, such information representing a signal to the farmer. The signal indicates that the sow of a given farrowing pen at the top of the list is not, compared to the other sows, for the intervals concerned, able to nourish its piglets to a desired degree.

An apparatus including the CRTU 3 and its associated connected components represented in the drawing is optionally designed to function in a farrowing pen with dust and ammonia vapours. Therefore, the apparatus beneficially fulfils requirements for IP 55 and is made with components fabricated from stainless and/or acid-proof materials. The CRTU 3 can be implemented using a programmable read only memory (PROM), a programmable logic circuit (PLC) or a personal computer (PC) or other computer based solution.

The invention is susceptible to being used in conjunction with various species of animals in a situation:
(a) wherein at least one young animal is provided with a special pen-zone;
(b) wherein a certain climate is provided fulfilling a secondary need of the at least one young animal; and
(c) wherein the at least one young animal will seek to the pen-zone, especially after having at least partly fulfilled its primary need for nourishment.

This specific climate may be achieved by cooling as well as by heating. The invention is particularly elucidated In connection with boxes or nests for the young piglets of a sow in a farrowing pen and in connection with the sow and its piglets in the first four to five weeks the piglets' life. By using the method and associated apparatus disclosed in the foregoing, a farmer is able to take appropriate actions based on one or more signals generated by the apparatus implementing the method. These actions may for instance be changing the nourishment provided to a sow, replacing piglets from one sow to another sow, providing treatment of inflammation of sows' udders or providing treatment for farrow fever of the sow, so as to provide improved living conditions for at least the sow and its piglets in farrowing pens wherein the sow is tethered. However, the method and associated apparatus are also applicable to farrowing pens whose sows are untethered.

It will be appreciated that embodiments of the invention described in the foregoing are susceptible to being modified without departing from the scope of the invention as defined by the accompanying claims.

In the accompanying claims, numerals and other symbols included within brackets are included to assist understanding of the claims and are not intended to limit the scope of the claims in any way.

Expressions such as "comprise", "include", "incorporate", "contain", "is" and "have" are to be construed in a non-exclusive manner when interpreting the description and its associated claims, namely construed to allow for other items or components which are not explicitly defined also to be present. Reference to the singular is also to be construed to be a reference to the plural and vice versa.

## Claims

1. A method of detecting a physical condition of health of a mother animal breastfeeding at least one corresponding young animal, said method comprising steps of:
- monitoring one or more movements of said at least one young within an area (1),
- collecting positional data of said at least one young animal,
- processing said positional data to generate data representative of movement patterns of said at least one young, and
- analysing said data representative of movement patterns of said at least one young animal to determine the physical condition of health of said at least one young animal and/or said mother animal.

2. A method as claimed in claim 1, wherein said analysing step comprises a further step of:
- comparing said data representative of movement patterns of said at least one young animal with a reference movement pattern,
wherein said reference movement pattern is a movement pattern of a young animal during its time of life when breastfeeding by its mother animal when in a healthy state, or a movement pattern of said young animal corresponding to another time interval.

3. A method as claimed in claim 1 or claim 2, said method further comprising steps of:
- dividing said area (1) being monitored into at least two zones, namely a feeding zone and a nesting zone, the feeding zone being a region wherein said at least one young animal is breastfed;
- comparing said movement patterns with said reference movement pattern to detect durations and frequencies said at least one young animal spends in at least one of said zones, and
wherein the durations and frequencies said at least one young animal spends in the at least one zone are indicative of the physical condition of health of at least said mother animal.

4. A method as claimed in any one of claims 1 to 3, wherein the movement patterns are determined by measuring at least one value within the area (1), said value being correlated to said at least one young animal during its time of life when breastfed by said mother animal.

5. A method as claimed in any one of claims 1 to 4, where the movement patterns are determined by measuring at least one value of a temperature within the nesting zone.

6. A method as claimed in any one of claims 1 to 4, further comprising a step of determining the movement patterns by measuring at least one value of power supplied to a heater arrangement (2) operable to heat the nesting zone.

7. A method as claimed in any one or claims 1 to 4, wherein the movement patterns are determined by measuring at least one value of an infrared surface temperature of a surface within the nesting zone.

8. An apparatus for detecting a physical condition of health of a mother animal during its time of life when breastfeeding at least one corresponding young animal, said apparatus comprising
- a temperature measuring arrangement for measuring at least one temperature of a nesting zone, said nesting zone being disposed for receiving said at least one young animal;
- a processing and analyzing arrangement for processing and analyzing said at least one temperature measured by said temperature measurement arrangement, and for detecting a duration and frequency of said at least one young in said nesting zone and generating a corresponding output,
- a determination arrangement for determining from said output from said processing and analyzing arrangement at least one physical condition of health of said mother animal during its time of life when breastfeeding said at least one young or of said at least one young animal, and
- a signal generating arrangement coupled to said determining arrangement for providing at least one signal in response to said determination for providing an alarm indicative of a physically unhealthy condition of the mother animal and/or said at least one young animal.

9. An apparatus for detecting a physical condition of health of a mother animal during its time of life when breastfeeding at least one young corresponding animal, said apparatus comprising
- a power measuring arrangement provided for measuring power supplied to a heating device of a nesting zone, said nesting zone being disposed for accommodating said at least one young animal,
- a processing and analyzing arrangement for processing and analyzing said power measurement, for detecting a duration and frequency said at least one young is in the nesting zone,
- a determining arrangement for determining, based on the said detection, at least one physical condition of health of said mother animal during its time of life when breastfeeding said at least one young animal or of said at least one young animal being breastfed by the mother animal; and
- a signal generating arrangement coupled to said determining arrangement for providing at least one signal in response to said determination for providing an alarm indicative of a physically unhealthy condition of the mother animal and/or at least one young animal.

10. An apparatus as claimed in claim 8, wherein the temperature measurement arrangement includes an infrared temperature-measuring device (5) provided for measuring a surface temperature in the nesting zone.
